# EUROPEAN PATENT APPLICATION

(11) **EP 0 617 046 A1**
(43) Date of publication of application: **28.09.1994**
(21) Application number: 94104307.7
(22) Date of filing: 18.03.1994
(51) Int. Cl.: C07H 19/02, C07H 19/067, C07H 19/167, A61K 31/70

(54) **Nucleoside derivatives and antiherpes composition**

(30) Priority: 18.03.1993 JP 84044/93; 27.10.1993 JP 292617/93
(71) Applicant: NIPPON CHEMIPHAR CO., LTD., Chiyoda-ku Tokyo101 (JP)
(72) Inventor: Hata, Tsujiaki, Kawasaki-shi, Kanagawa (JP); Ishikawa, Masahide, Yokohama-shi, Kanagawa (JP); Masaki, Mitsuo, Chiba-shi, Chiba (JP); Matsuzaki, Katsuhiro, Chiba-shi, Chiba (JP)
(74) Representative: Kraus, Walter, Dr.

(57) **Abstract**

Disclosed is a novel nucleoside derivative showing anti-virus activities such as an anti-herpes activity and an anti-HIV activity. The nucleoside derivative has the formula (1-1) or (1-2): 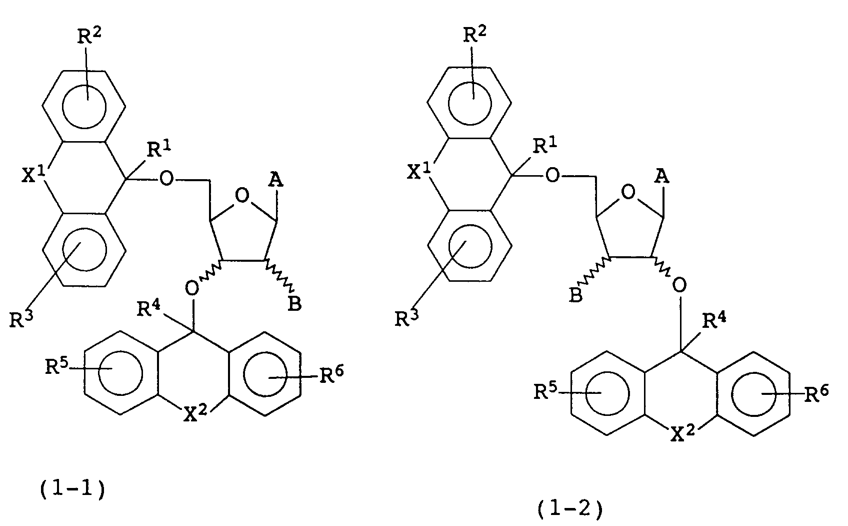
wherein A is a nitrogen atom-containing heterocyclic ring which is linked through nitrogen atom thereof; B is a hydrogen atom, a halogen atom, an azido group, an alkyl group, etc.

## Description

### BACKGROUND OF THE INVENTION

### Field of Invention

The present invention relates to novel nucleoside derivatives, a process for the preparation of the same, and an anti-herpes composition containing the same.

### Description of Prior Art

Recently, studies for providing medicaments for treating various virus diseases have been earnestly made. Particularly, acquired immune deficiency syndrome (AIDS) constitutes a serious social problem. It is known that AIDS is caused by infection with human immunodeficiency virus which is one of retroviruses. At present, 3'-deoxy-3'-azidothymidine (AZT) is approved as a medicament for treating the HIV infectious disease. However, this medicament has a problem that it shows high toxicity to host cells so as to cause troublesome side effect. Therefore, development of compounds showing high anti-retrovirus activity but low toxicity to host cells has been desired.

Also desired is a compound showing high anti-herpes virus activity, particularly anti-varicella-zoster virus activity. As an anti-herpes virus medicament, Acyclovir which is a guanosine derivative is known. Acyclovir shows inhibitory effect to herpes simplex virus (HSV) type 1 and type 2 at a low concentration of less than 0.05 µL/mL, while it shows inhibitory effect to varicella-zoster virus (VZV) at an extremely high concentration such as 400 to 600 times as much as that for HSV. Moreover, it is reported that Acyclovir is not effective to cytomegalovirus (CMV) having no thymidine kinase (TK) in "Clinic and Study, vol. 70, No.8 (1993, August), page 21". Varicella-zoster virus strain is classified into two types, that is, thymidine kinase-positive (TK⁺) strain and thymidine kinase-deficient (TK⁻) strain. Accordingly, an anti-herpes agent having activity to varicella-zoster virus strains of both types, particularly the thymidine kinase-deficinet varicella-zoster virus is desired.

### SUMMARY OF THE INVENTION

An object of the present invention is to provide a new nucleoside derivative having high antiviral activities such as high anti-herpes virus activity and high anti-retrovirus activity.

Specifically, the invention has an object to provide a new nucleoside derivative having high antiviral activities to human immunodeficiency virus and varicella-zoster virus.

The present invention resides in a nucleoside derivative having the formula (1-1) or (1-2): 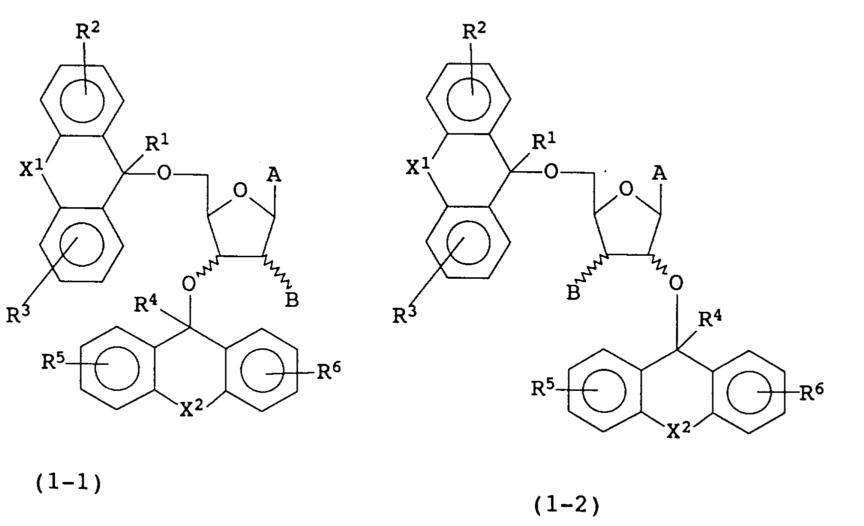
wherein A is a nitrogen atom-containing heterocyclic ring which is linked through nitrogen atom thereof; B is a hydrogen atom, a halogen atom, an azido group, an alkyl group having 1-7 carbon atoms, an aralkyl group having 5-19 carbon atoms, an alkoxyalkyl group having 2-15 carbon atoms, an alkoxy group having 1-7 carbon atom, a haloalkoxy group having 1-7 carbon atoms, an aralkyloxy group having 5-19 carbon atoms, an aryloxy group having 4-10 carbon atoms, a cycloalkyloxy group having 3-7 carbon atoms, or a cycloalkyloxy group having 4-6 carbon atoms and one or two heteroatoms selected from the group consisting of oxygen, nitrogen and sulfur; each of R¹ and R⁴ independently is a hydrogen atom, an alkyl group having 1-7 carbon atoms, a cycloalkyl group having 3-7 carbon atoms, an aralkyl group having 5-19 carbon atoms, or an aryl group having 4-10 carbon atoms, in which each of the aralkyl group and aryl group may have one or more substituents selected from the group consisting of a halogen atom, an alkyl group having 1-7 carbon atoms, an alkoxy group having 1-7 carbon atoms, a haloalkyl group having 1-7 carbon atoms, an alkoxyalkyl group having 2-15 carbon atoms and a haloalkoxy group having 1-7 carbon atoms; each of R², R³, R⁵ and R⁶ independently is a hydrogen atom, a halogen atom, a hydroxyl group, an alkyl group having 1-7 carbon atoms, an alkoxy group having 1-7 carbon atoms, a haloalkyl group having 1-7 carbon atoms, an alkoxyalkyl group having 2-15 carbon atoms, a haloalkoxy group having 1-7 carbon atoms, an aralkyl group having 5-19 carbon atoms, or an aryl group having 4-10 carbon atoms; and X¹ and X² independently is an oxygen atom, a sulfur atom, a methylene group or a carbonyl group.

The nucleoside derivatives of the formulas (1-1) and (1-2) can be in the form of their pharmaceutically acceptable salts.

In the formulas (1-1) and (1-2), the steric configuration on the 2nd and 3rd positions in the saccharide ring can be in the arabino type, xylo type, ribo type or lyxo type, of deoxy type thereof.

### DETAILED DESCRIPTION OF THE INVENTION

The "A" in the formulas (1-1) and (1-2) is a heterocyclic ring containing at least one nitrogen atom as its ring member and is connected to the saccharide ring in the 1st position through its nitrogen atom. Preferred structures of the nitrogen-containing heterocyclic group represented by "A" are of a single ring having at least two nitrogen atoms as the ring members and of a condensed double ring having at least two nitrogen atoms as the ring members of each ring.

Preferred examples of the "A" of the single ring type having at least two nitrogen atoms are the groups of the following formulas (5-1) to (5-4): 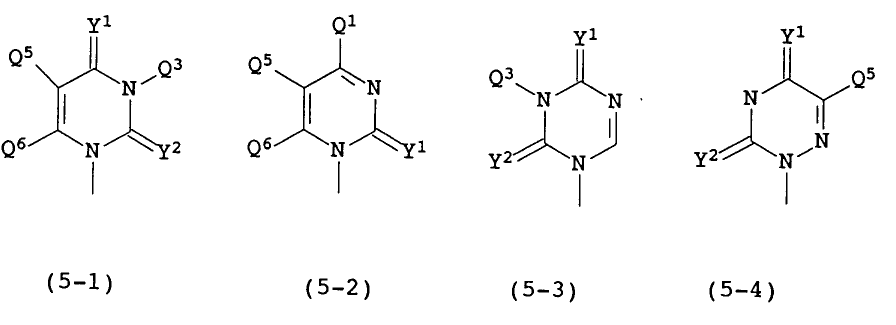
wherein
Q¹ is a hydrogen atom, a halogen atom, a group represented by -NQ⁷Q⁸, a group represented by -N=CQ⁹Q¹⁰_{,} or a group represented by -OQ⁷, wherein each of Q⁷ and Q⁸ independently is a hydrogen atom, an alkyl group having 1-7 carbon atoms (preferably 1-4 carbon atoms), a haloalkyl group having 1-7 carbon atoms (preferably 1-4 carbon atoms), an aralkyl group having 5-19 carbon atoms, an aryl group having 4-10 carbon atoms (preferably 4-6 carbon atoms), an acyl group having 1-10 carbon atoms, an allyl group, or a group having the formula (6): 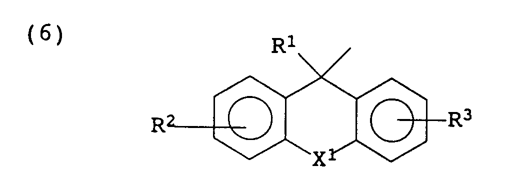
wherein R¹ is a hydrogen atom, an alkyl group having 1-7 carbon atoms (preferably 1-4 carbon atoms), a cycloalkyl group having 3-7 carbon atoms, an aralkyl group having 5-19 carbon atoms, or an aryl group having 4-10 carbon atoms, wherein the aralkyl group and aryl group may have one or more substituents selected from the group consisting of a halogen atom, an alkyl group having 1-7 carbon atoms (preferably 1-4 carbon atoms), an alkoxy group having 1-7 carbon atoms (preferably 1-4 carbon atoms), a haloalkyl group having 1-7 carbon atoms (preferably 1-4 carbon atoms), and a haloalkoxy group having 1-7 carbon atoms (preferably 1-4 carbon atoms); each of R² and R³ independently is a hydrogen atom, a halogen atom, a hydroxyl group, an alkyl group having 1-7 carbon atoms (preferably 1-4 carbon atoms), an alkoxy group having 1-7 carbon atoms (preferably 1-4 carbon atoms), a haloalkyl group having 1-7 carbon atoms (preferably 1-4 carbon atoms), a haloalkoxy group having 1-7 carbon atoms (preferably 1-4 carbon atoms), an aralkyl group having 5-19 carbon atoms, or an aryl group having 4-10 carbon atoms; and X¹ is an oxygen atom, a sulfur atom, a methylene group, or a carbonyl group, and Q⁹ and Q¹⁰ independently is a hydrogen atom, an alkyl group having 1-7 carbon atoms (preferably 1-4 carbon atoms), an alkoxy group having 1-7 carbon atoms (preferably 1-4 carbon atoms), a haloalkyl group having 1-7 carbon atoms (preferably 1-4 carbon atoms), an aralkyl group having 5-19 carbon atoms, an aryl group having 4-10 carbon atoms, an allyl group, or a group represented by -NQ⁷Q⁸ wherein each of Q⁷ and Q⁸ has the same meaning as above;
Q³ is a hydrogen atom, an alkyl group having 1-7 carbon atoms (preferably 1-4 carbon atoms), a haloalkyl group having 1-7 carbon atoms (preferably 1-4 carbon atoms), an aralkyl group having 5-19 carbon atoms, an acyl group having 1-10 carbon atoms, an aryl group having 4-10 carbon atoms, or an allyl group;
Q⁵ is a hydrogen atom, a halogen atom, an alkyl group having 1-7 carbon atoms (preferably 1-4 carbon atoms), a haloalkyl group having 1-7 carbon atoms (preferably 1-4 carbon atoms), an aralkyl group having 5-19 carbon atoms, an aryl group having 4-10 carbon atoms, or an allyl group;
Q⁶ is a hydrogen atom, a halogen atom, a thio group, or a seleno group, wherein the thio group and seleno group may have a substituent selected from the group consisting of an alkyl group having 1-7 carbon atoms (preferably 1-4 carbon atoms), a haloalkyl group having 1-7 carbon atoms (preferably 1-4 carbon atoms), an aralkyl group having 5-19 carbon atoms, an aryl group having 4-10 carbon atoms, or an allyl group; and
each of Y¹ and Y² independently is an oxygen atom or a sulfur atom.

Preferred examples of the "A" of the condensed ring type having at least two nitrogen atoms are the groups of the following formulas (7-1) to (7-13): 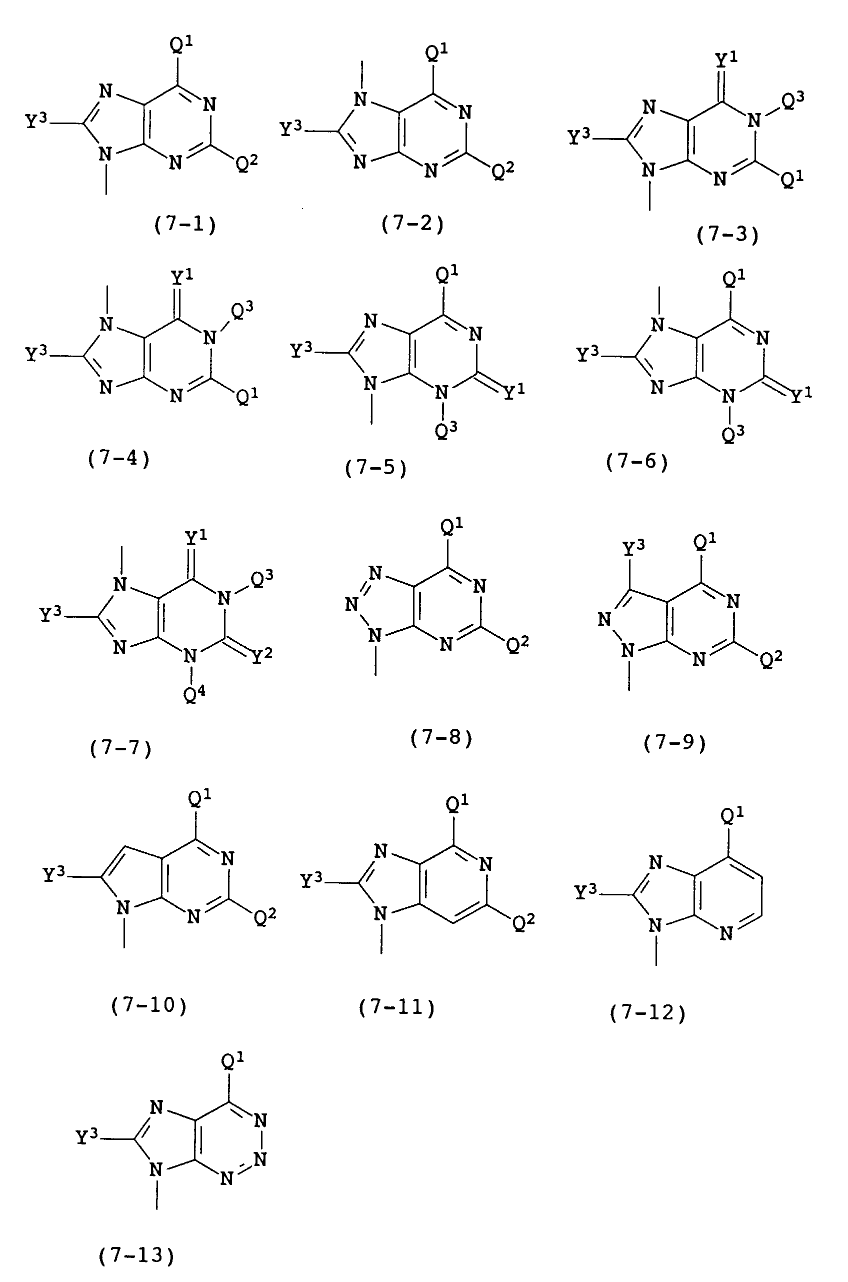
wherein each of Q¹, Q³, Y¹, and Y² has the same meaning as defined above, Q² has the same meaning as that of Q¹, Q⁴ has the same meaning as that of Q³, Y³ is a hydrogen atom, a halogen atom, or a hydroxyl group.

The halogen atom preferably is fluorine, chlorine, or bromine, except for the halogen atom for "Z" which is seen afterwards in the specification.

Preferred examples of the alkyl group having 1-7 carbon atoms (preferably 1-4 carbon atoms) include methyl, ethyl, propyl, isopropyl, n-butyl, sec-butyl, and t-butyl.

Preferred examples of the alkoxy group having 1-7 carbon atoms (preferably 1-4 carbon atoms) include methoxy, ethoxy, propoxy, isopropoxy, and n-butoxy.

Preferred examples of the haloalkyl group having 1-7 carbon atoms (preferably 1-4 carbon atoms) include trifluoromethyl and 2,2,2-trifluoroethyl.

Preferred examples of the haloalkoxy group having 1-7 carbon atoms (preferably 1-4 carbon atoms) include trifluoromethoxy and 2,2,2-trifluoroethoxy.

Preferred examples of the alkoxyalkyl group having 2-15 carbon atoms (preferably 2-8) include methoxymethyl, ethoxymethyl, methoxyethyl, and methoxybutyl.

Preferred examples of the aralkyl group having 5-19 carbon atoms are aralkyl groups having 7-19 carbon atoms and an aromatic hydrocarbon ring such as phenyl or naphthyl, and aralkyl groups having 5-19 carbon atoms and a heterocyclic ring such as 2-furyl, thienyl, or pyridyl. Particularly preferred are benzyl, phenethyl, trityl, furfuryl, and thenyl.

Preferred examples of the aralkyloxy group having 5-19 carbon atoms are aralkyloxy groups having 7-19 carbon atoms and an aromatic hydrocarbon ring and aralkyl groups having 5-19 carbon atoms, and a heterocyclic ring. Particularly preferred are benzyloxy, phenethyloxy, trityloxy, furfuryloxy, and thenyloxy.

Preferred examples of the cycloalkyl group having 3-7 carbon atoms include cyclopyropyl and cyclohexyl.

Preferred examples of the cycloalkyloxy group having 3-7 carbon atoms include cyclopyropyloxy and cyclohexyloxy.

Preferred examples of the aryl group having 4-10 carbon atoms are aromatic hydrocarbon groups having 6-10 carbon atoms and aromatic heterocyclic groups having 4-10 carbon atoms. Particularly preferred are phenyl, naphthyl, 2-furyl, thienyl, and pyridyl.

Preferred examples of the aryloxy group having 4-10 carbon atoms are aryloxy group having aromatic hydrocarbon groups of 6-10 carbon atoms and aryloxy group having aromatic heterocyclic groups of 4-10 carbon atoms. Particularly preferred are phenoxy, naphthoxy, tolyloxy, 2-furyloxy, and thienyloxy.

Preferred examples of the acyl group having 1-10 carbon atoms include formyl, acetyl, propionyl, benzoyl, and methoxybenzoyl.

If the cycloalkyloxy group having 4-6 carbon atoms (preferably 4 or 5) and one or two heteroatoms selected from the group consisting of oxygen, nitrogen and sulfur has a nitrogen atom as its ring member, the nitrogen atom can have an alkyl group having 1-7 carbon atoms (preferably 1-4 carbon atoms) or an aralkyl group having 5-19 carbon atoms (preferably 5-7 carbon atoms) as a substituent.

Particularly preferred groups for the "A" in the formulas (1-1) and (1-2) include those represented by the following formulas (8-1) to (8-27): 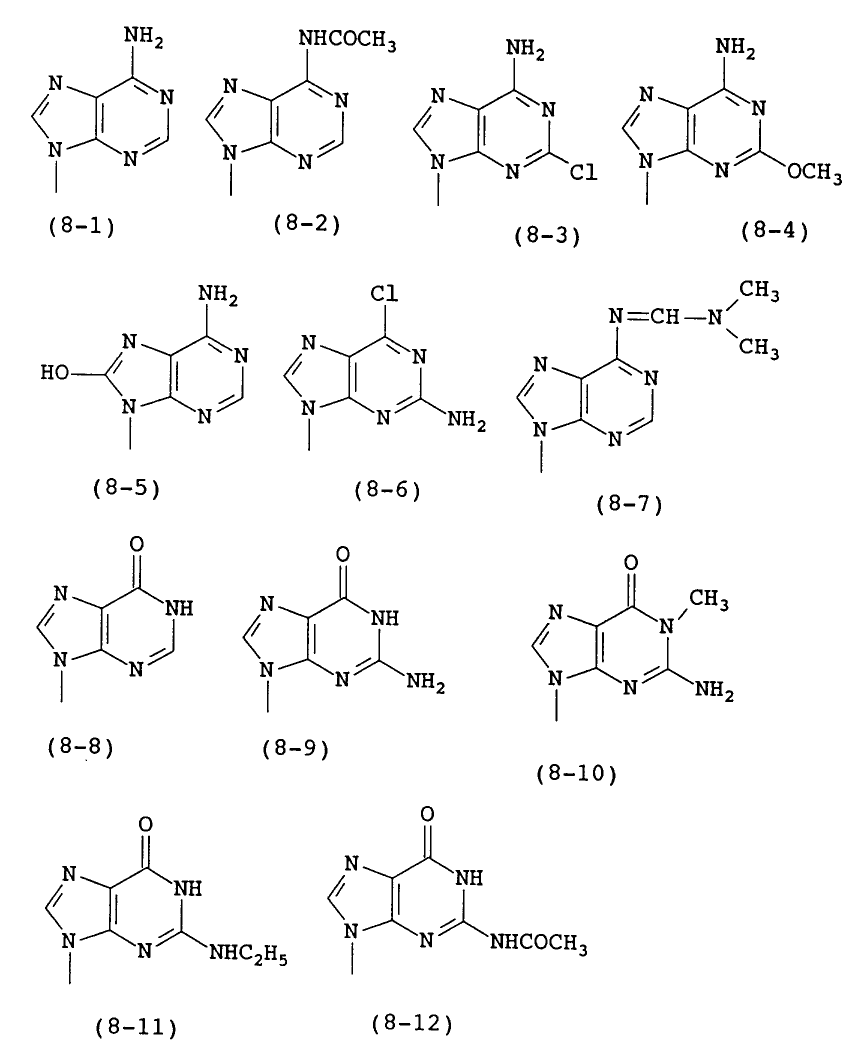 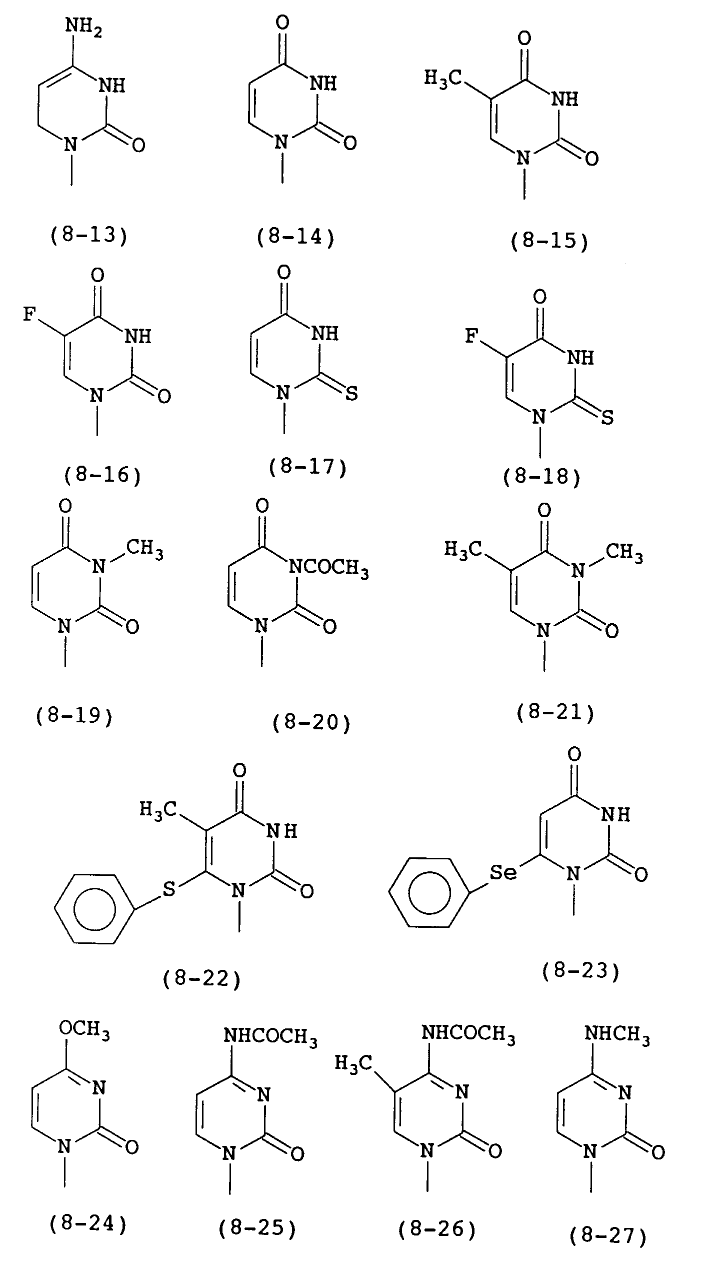
The "B" in the formulas (1-1) and (1-2) preferably is hydrogen, methoxy, ethoxy, phenoxy, trifluromethoxy, aralkyloxy (e.g., benzyloxy), halogen (e.g., fluorine, chlorine, and bromine), azido, or one of the following formulas (9-1) to (9-6): 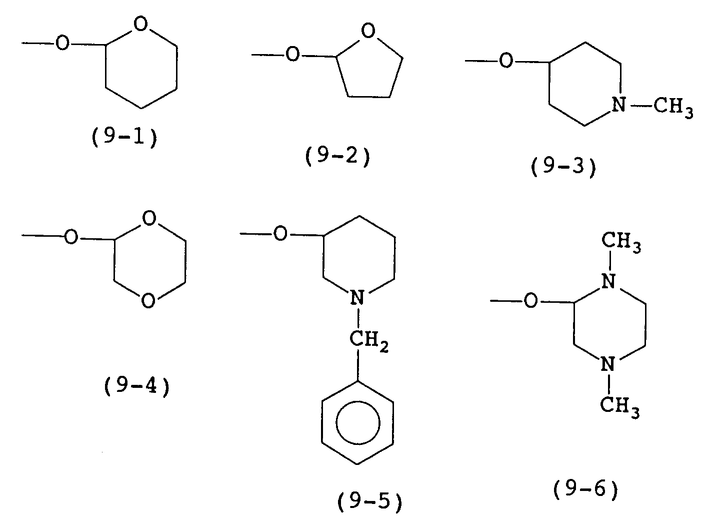
Particularly preferred examples of the aralkyl group of 5-19 carbon atoms and aryl group of 4-10 carbon atoms which have one or more substituents (listed for R¹ and R⁴) include 3,4-dimethoxyphenethyl, 4-methoxyphenyl, tolyl, 3-chlorophenyl, 3,4,5-trimethoxyphenyl, and 4-bromofuryl.

The groups represented by the following formulas (10-1) and (10-2): 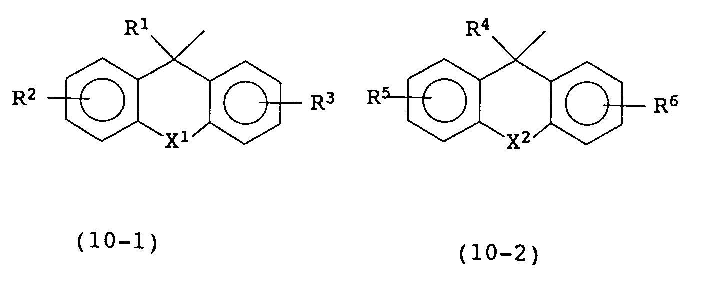
in the formulas (1-1) and (1-2) preferably are the following groups: 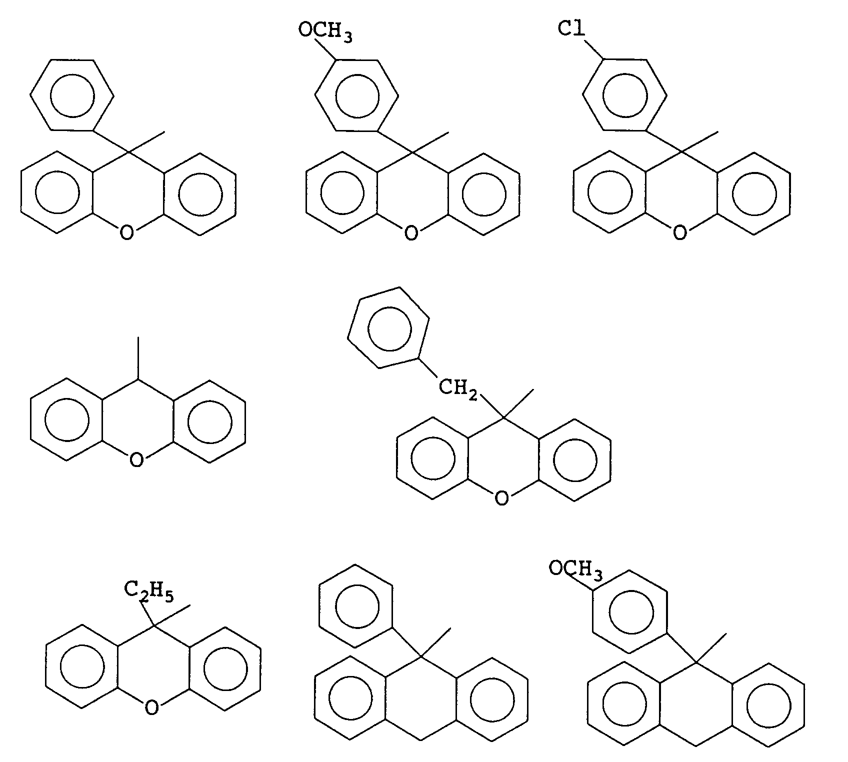
The pharmaceutically acceptable salt of the nucleoside derivative of the formula (1-1) or (1-2) are prepared from the nucleoside and an inorganic or organic acid or a base. Examples of the acid salt include hydrochloride, sulfate, acetate, tartrate, fumarate, mesylate, and tosylate. Examples of the base salt include sodium salt and potassium salt.

Processes for preparing the nucleoside derivatives and their pharmaceutically acceptable salts of the invention are described below.

The nucleoside derivative and its pharmaceutically acceptable salt having the formula (1-1) or (1-2) wherein R¹ and R⁴ are the same, R² and R⁵ are the same, and R³ and R⁶ are the same, namely, a nucleoside derivative of the formula (4-1) or (4-2): 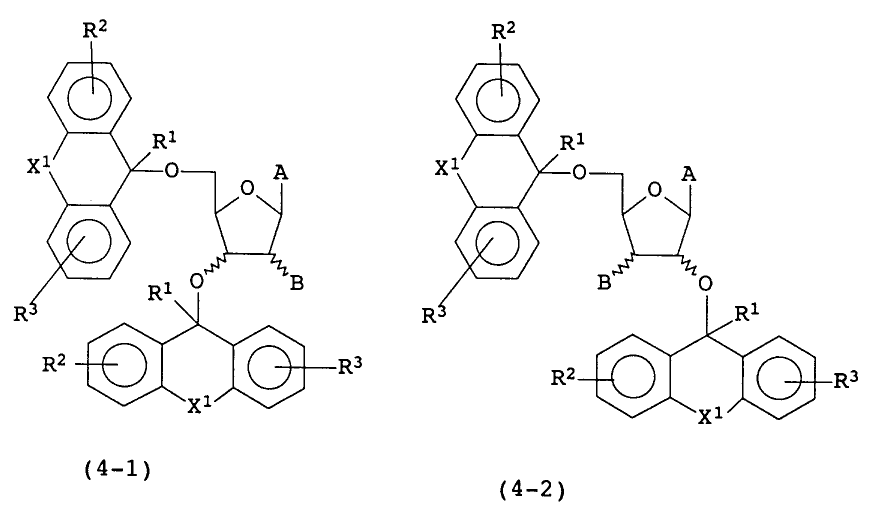
can be prepared by a process which comprises causing a condensation reaction between a nucleoside derivative having the formula (2-1) or (2-2): 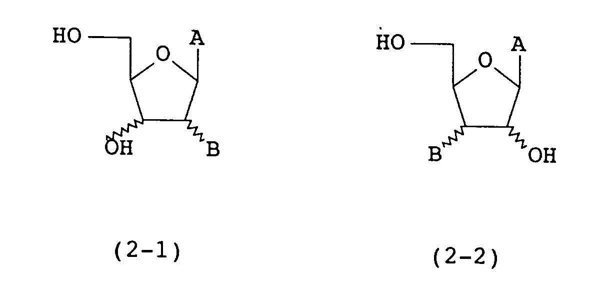
and a compound having the formula (3): 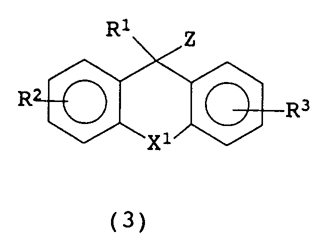
wherein each of R¹, R², R³, and X¹ has the same meaning as defined in claim 1, and Z is a halogen atom or a sulfonyloxy group.

If necessary, the protective group which may be attached to the group of "A" in the formula (4-1) or (4-2) can be removed or replaced with other protective group.

In the formula (3), the halogen atom for Z preferably is chlorine, bromine, or iodine. The sulfonyloxy group preferably is methanesulfonyloxy, p-toluenesulfonyloxy, or trifluoromethanesulfonyloxy.

In the reaction, the nucleoside derivative of the formula (2-1) or (2-2) [referred to hereinbelow as compound (2)] and the compound of the formula (3) [referred to hereinbelow as compound (3)] are preferably employed under the conditions that approx. 1-20 equivalent amount (preferably 2-10 equivalent amount) of the compound (3) is employed per one equivalent amount of the compound (2).

The reaction of the compound (2) with the compound (3) can be performed in an organic solvent in the presence or absence of a base. In the case of using the base, the base preferably is potassium carbonate, lithium hydride, sodium hydride, pyridine, or triethylamine. The base can be used in an amount of not more than 2 equivalents, preferably 0.5-1.5 equivalents, more preferably 0.8-1.2 equivalents, per one equivalent amount of the compound (3).

There is no specific limitation with respect to the reaction solvent, so long as the solvent is inert to the reaction. Ethyl ether, benzene, chloroform, acetone, N,N-dimethylformamide (DMF), dimethylsulfoxide (DMSO), 1,3-dimethyl-2-imidazoline, hexamethylphosphorictriamide (HMPA), and pyridine are preferably employed. The reaction can be performed at a temperature ranging from 0°C to the reflux temperature of the solvent employed, preferably from room temperature to 170°C. The incorporation or removal of the protective group, and the formation of the pharmaceutically acceptable salt of the resulting nucleoside derivative can be performed in a known manner. The compound (2) and compound (3) are known compounds, or can be prepared from a known compound in a known manner.

The nucleoside derivative of the formula (1-1) or (1-2) can be prepared also by one of the following processes (A), (B), (C), (D-1), and (D-2).

### Process (A)

A nucleoside derivative having one of the following formulas (12-1) and (12-2): 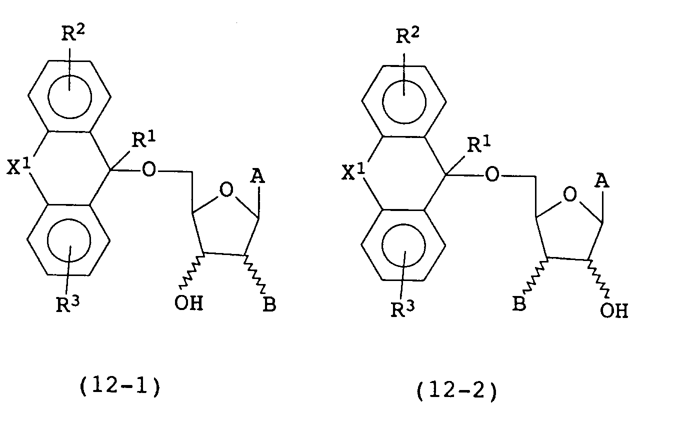
wherein each of A, B, R¹, R², R³, and X¹ has the same meaning as defined for the formulas (1-1) and (1-2), is caused to react under condensation conditions with a compound having the formula (13): 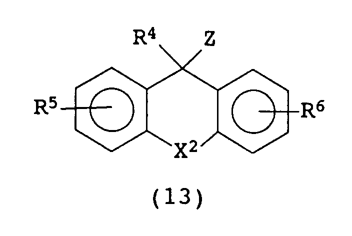
wherein each of R⁴, R⁵, R⁶, and X² has the same meaning as defined for the formulas (1-1) and (1-2), and Z has the same meaning as defined for the formula (3).

In the reaction, the nucleoside derivative of the formula (12-1) or (12-2) [referred to hereinbelow as compound (12)] and the compound of the formula (13) [referred to hereinbelow as compound (13)] are preferably employed under the conditions that approx. 0.5-10 equivalent amount (preferably 1-5 equivalent amount) of the compound (13) is employed per one equivalent amount of the compound (12).

Other conditions described for the reaction of the process for preparing the nucleoside derivative of the formula (4-1) or (4-2) can be employed for the reaction of the process (A).

### Process (B)

A nucleoside derivative having one of the following formulas (14-1) and (14-2): 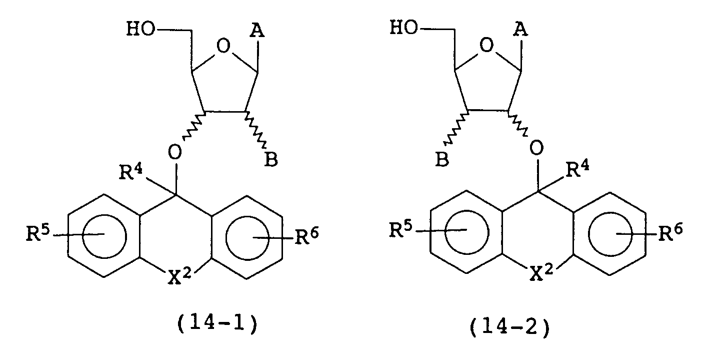
wherein each of A, B, R⁴, R⁵, R⁶, and X² has the same meaning as defined for the formulas (1-1) and (1-2), is caused to react under condensation conditions with a compound having the aforementioned formula (3).

In the reaction, the nucleoside derivative of the formula (14-1) or (14-2) [referred to hereinbelow as compound (14)] and the compound of the formula (3) [compound (3)] are preferably employed under the conditions that approx. 0.5-10 equivalent amount (preferably 1-5 equivalent amount) of the compound (3) is employed per one equivalent amount of the compound (14).

Other conditions described for the reaction of the process for preparing the nucleoside derivative of the formula (4-1) or (4-2) can be employed for the reaction of the process (B).

### Process (C)

A nucleoside derivative having one of the following formulas (15-1) and (15-2): 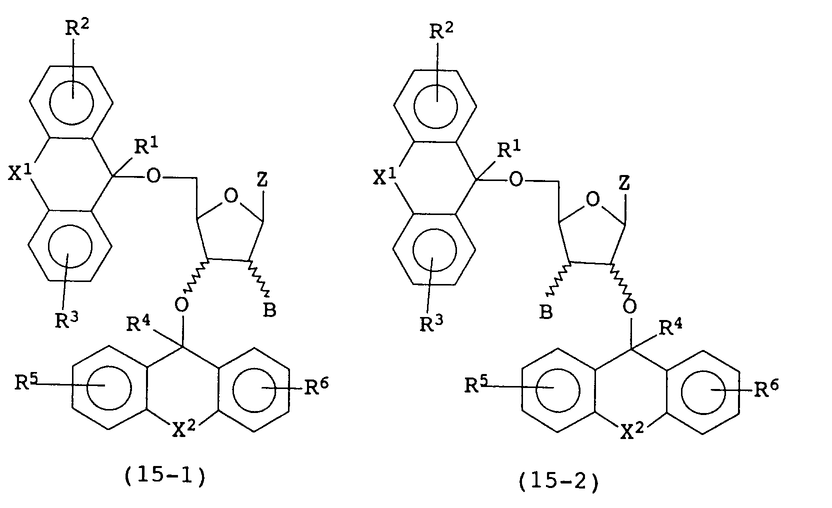
wherein each of B, R¹, R², R³, R⁴, R⁵, R⁶, X¹, and X² has the same meaning as defined for the formulas (1-1) and (1-2), and Z has the same meaning as defined for the formula (3) or represents acetoxy.
is caused to react under condensation conditions with a compound having the formulas (16-1), (16-2), and (16-3):

A-H (16-1)

A-M (16-2)

A-Si(CH₃)₃ (16-3)

wherein A has the same meansing as defined for the formulas (1-1) and (1-2), and M is a monovalent metal, preferably Na, K, Ag, Hg, and HgCl.

In the reaction, the nucleoside derivative of the formula (15-1) or (15-2) [referred to hereinbelow as compound (15)] and the compound of the formula (16-1), (16-2), or (16-3) [referred to hereinbelow as compound (16)] are preferably employed under the conditions that approx. 1-20 equivalent amount (preferably 1-10 equivalent amount) of the compound (16) is employed per one equivalent amount of the compound (15).

The reaction between the compound (15) having a halogen atom as the "Z" and a compound of the formula (16-1) can be performed, for instance, in nitromethane in the presence of mercury cyanide. Reference is made to J. Org. Chem., 30, 149 (1965).

The reaction between the compound (15) having a halogen atom as the "Z" and a compound of the formula (16-2) can be performed in an inert solvent (e.g., benzene, toluene, xylene, acetonitrile, or dichloromethane). Reference is made to J. Am. Chem.Soc., 78, 2117 (1956).

The reaction between the compound (15) having a halogen atom as the "Z" and a compound of the formula (16-3) can be performed in the manner described in J. Org. Chem., 34, 431 (1969).

The reaction between the compound (15) having acetoxy as the "Z" and a compound of the formula (16-1) can be performed, for instance, in 1,2-dichloroethane in the presence of tin(IV) chloride or aluminium chloride. Reference is made to J. Org. Chem., 39, 3654, 3660, 3664, 3668 (1974). This reaction also can be performed at 130-195°C under reduced pressure in the presence or absence of a Lewis acid. Reference is made to Bull. Chem. Soc. Japan., 46, 556, 3858 (1973).

### Process (D-1)

A nucleoside derivative having one of the following formulas (17-1) and (17-2): 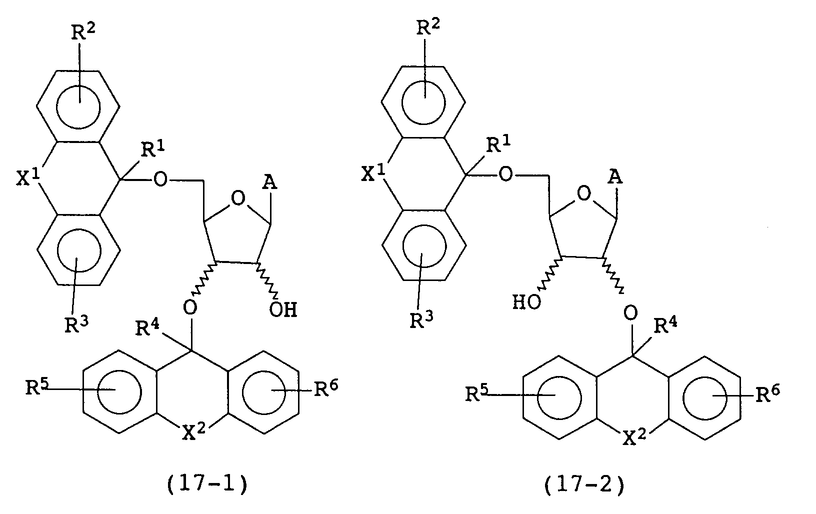
wherein each of A, R¹, R², R³, R⁴, R⁵, R⁶, X¹, and X² has the same meaning as defined for the formulas (1-1) and (1-2),
is caused to react with the aforementioned alkyl, aryl, haloalkyl or cycloalkyl compound having the releasing group "Z" to give a nucleoside derivative of the formula (1-1) or (1-2) in which B is alkyloxy, aryloxy, haloalkyloxy, or cycloalkyloxy.

### Process (D-2)

The nucleoside derivative of the formulas (17-1) and (17-2) (referred to as compound (17)) and a cycloalkenyl compound having one or two oxygen, nitrogen or sulfur atoms as ring members are subjected to addition reaction to give a nucleoside derivative of the formula (1-1) or (1-2) in which B is an cycloalkyloxy group having 4-6 carbon atoms and one or two oxygen, nitrogen or sulfur atoms as ring members.

### Process (E)

A nucleoside derivative having one of the following formulas (18-1) and (18-2): 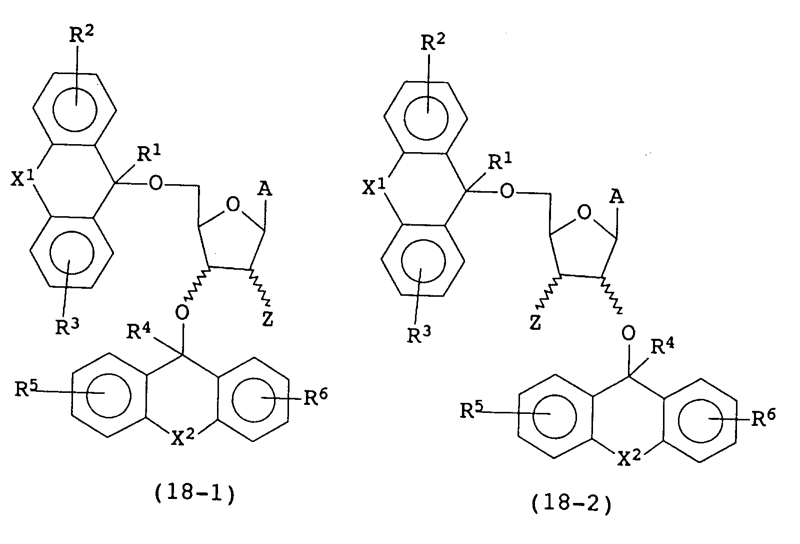
wherein each of A, R¹, R², R³, R⁴, R⁵, R⁶, X¹, and X² has the same meaning as defined for the formulas (1-1) and (1-2) and Z has the meaning as defined for the formula (3), is caused to react with an appropriate azide-forming agent (e.g., lithium azide) or a fluorinating agent (e.g., tetraalkylammonium fluoride such as tetrabutylammonium fluoride) to give a nucleoside derivative of the formula (1-1) or (1-2) in which B is an azido group or a fluorine atom).

The starting materials employed for the above-stated reactions are described, for instance, J. Am. Chem., Soc., 106(16) 1984, p 4552, Nucleoic Acids Research, 17(20) 1989, p 8135, Nucleosides & Nucleotides, 11(7) 1992, p 1333, Biochemistry, 30(40), p 9735, Biochemistry, 30(16), p 4001, Tetrahedron Lett., 22(45), p 4537, etc.

The HIV growth inhibition by the nucleoside derivative of the invention is examined by the following HIV growth inhibition tests.

### 1. Culture

A solution in which 1.2 x 10⁵ cells are suspended is prepared by culturing H9 cell (originating from human T lymphocyte) of 2.0 x 10⁵ in a medium for 24 hours, centrifuging the cultured medium, suspending the cell of 1.0 x 10⁶ for 30 min., and diluting the suspended cell.

A tested compound is dissolved in DMSO (dimethylsulfoxide) at different concentrations, and 12.5 µL of each solution is placed on each hole of a 24-well plate.

In each well having received the tested compound, 2,000 µL of the 1.2 x 10⁵ cell suspension is placed. The plate is covered and shaken vigorously to mix the suspensions, and the plate is allowed to stand at 37°C for 1 hour. Subsequently, 50 µL of a preserved high titer HIV solution is placed in each well, and the content in the well is mixed using a 1-mL pipette. The mixtures in the wells are cultured at 37°C in 5% CO₂ atmosphere. On day 4 of the culture, the cultured mixture is split into two portions. One portion is subjected to measurement of living cells by MTT method at day 7 of the culture. From another portion of the mixture, a sample of 1,000 µL is taken and mixed with a fresh medium of the same volume. The culture is then continued using the resulting mixture up to day 7 days of the culture. Then, RT assay is conducted.

As positive control compound, azidothymidine (AZT) dissolved in DMSO and diluted to give a test solution having the same concentration as the above tested compound is used. Further, DMSO, H9 cell-suspension, and a suspension containing H9 cell and HIV are tested as controls.

### 2. MTT Assay for measurement of living cells

On the day 7 the culture, 100 µL of each sample is taken from one of the split cultured mixtures and placed on a new 96 well plate. To the cultured mixture in the well, 20 µL (2.5 mg/mL PBS) of MTT [3-(4,5-dimethylthiazol-2-yl)-2,5-diphenyltetrazolium bromide]-phosphoric acid buffer reagent (Sigma, No.M-2128) is added, and the resulting mixture is cultured at 37°C for 4 hours. To the cultured mixture is added 100 µL of isopropanol/0.04N-HCl, and the resulting mixture is mixed by frequent pipetting. Within one hour, absorbance is measured at 570 nm (reference: at 630 nm).

A standard curve is prepared from MTT data, and number of cells in each sample is calculated. Further, a living cell ratio (%) is calculated using the following formula:

Living cell ratio (%) = [number of cells in sample portion/number of cells in H9(+HIV) portion] x 100

### 3. RT (Reverse Transcryptase) assay

The assay is performed in the known manner.

The virus growth ratio (%) is calculated using the following formula:

Virus growth ratio (%) = [radioactivity in sample portion/radioactivity in H9(+HIV) portion] x 100

- Reference:: Poiesz, B.J. et al., Proc. Natl. Acad. Sci. U.S.A. 77, 7415-7419 (1980)

2'-O-Tetrapyranyl-3',5'-O-di(9-phenylxanthenyl)-adenosine obtained in Exmaple 2 was subjected to the MTT assay and RT assay at different concentrations by the above-mentioned HIV growth inhibition test. The living cell ratio (%) and virus growth ratio (%) obtained from the test are set forth in Table 1.

**Table 1**

| Concentratin of Test Compound (mg/mL) | Living Cell Ratio (%) | Virus Growth Ratio (%) |
|---|---|---|
| 200 | 109 | 17 |
| 20 | 116 | 17 |
| 2 | 117 | 26 |
| 0.2 | 97 | 84 |
| 0 | 100 | 100 |

As is apparent from the above data, the nucleoside derivative of the invention gives high virus growth inhibition activity, while showing high living cell ratio.

Accordingly, the nucleoside derivative is effective to treatment or precaution of diseases caused by retrovirus, particularly HIV.

The nucleoside derivative of the invention is further examined in antivairal activity to varicella-zoster virus (VZV) and cytotoxicity by the following tests.

### 1. Cell

### Human embryonic lung fibroblast (HEL cell, ATCC CCL-137)

Culture: The cell is cultured in a minimum essential medium (MEM, Gibco) supplemented with 10% inactivated bovine fetal serum, 1% L-glutamine, and 0.3% sodium hydrogen carbonate.

### 2. Virus

### Varicella-zoster virus (VZV) strain

(a) Thymidine kinase-positive (TK⁺) strain OKA & YS
(b) Thymidine kinase-deficient (TK⁻) strain YS/R & 07/1

### 3. Assay for antiviral activity

Cells cultured on a 96-well microtiter plate are inoculated with the virus at an input of 20 plaque formation units (PFU). The cultured cells are incubated at 37°C for 2 hours. Subsequently, remaining virus is removed, and the incubated cells are again cultured in a MEM medium to which are added the test compound, 10% inactivated bovine fetal serum, 1% L-glutamine, and 0.3% sodium hydrogen carbonate in duplicate. After five days culture, the microtiter plate is fixed by ethanol, stained by Giemsa staining method, and microscopically observed for measurement of number of plaques formed. The results are indicated by IC₅₀ (i.e., concentration at which the plaque formation by virus is inhibited by 50%.

### 4. Assay for cytotoxicity

HEL cellls of 3 x 10³ cells/well are seeded into a 96 well microtiter plate and allowed to proliferate in a MEM medium containing 10% inactivated bovine fetal serum at 37°C for 24 hours. Subsequently, the medium is changed into a MEM medium containing different concentrations of the tested compound, and then the incubation is continued for another 3 days. When monolayer cells are grown to 70%, number of cells is countered by Coulter Counter. The cytotoxicity is indicated by CC₅₀ (i.e., concentration at which the cell growth is inhibited by 50%.).

2'-O-Tetrapyranyl-3',5-O-di(9-phenylxanthenyl)-adenosine obtained in Exmaple 2 was subjected to the measurement of antiviral activity and cytotoxicity assay. The IC₅₀ and CC₅₀ obtained from the assay are set forth in Table 2.

**Table 2**

| Test Compound | Antiviral Activity IC₅₀ (µg/mL) | | | | Cytotoxicity CC₅₀ (µg/mL) |
|---|---|---|---|---|---|
| | TK⁺VZV | | TK⁻VZV | | |
| | OKA strain | YS strain | 07/1 strain | YS/R strain | |
| Product of Example 2 | 2 | 1 | 0.8 | 2 | >50 |

As is apparent from the above data, the nucleoside derivative of the invention gives high antiviral activity to TK⁺VZV as well as TK⁻VZV, while showing high CC₅₀ value.

Accordingly, the nucleoside derivative is effective to treatment or precaution of infectious diseases caused by VZV.

The nucleoside derivatives of the formulas (1-1) and (1-2) and their pharmaceutically acceptable salts according to the present invention can be administered either orally or parenterally to patients to treat diseases caused by retrovirus such as HIV or herpes casued by varicella-zoster virus (VZV), etc. Local administration and rectal administration can be also employed. The dose may usually be about 0.2 mg/kg/day to 200 mg/kg/day. The adiministration can be done once or more a day. The dose may be either increased or decreased depending on the age, body weight, and other conditions of the patients.

A variety of preparation forms can be adopted. For instance, tablets, powder, granules, capsules, suppository, troche, syrup, emulsion, soft gelatin capsules, cream, gel, paste, spray, and injectable preparations can be utilized. For these preparations, carriers, excipients, and other known additives can be employed. As the carriers, solid carriers such as lactose, kaolin, sucrose, crystalline cellulose, starch, talc, agar, pectin, magnesium stearate, and lecithin can be mentioned. Also employable are liquid carriers such as glycerol, polyvinyl pyrrolidone, olive oil, ethanol, benzyl alcohol, propylene glycol and water.

The following examples illustrate typical synthetic processes for preparing the nucleoside derivatives of the invention.

### Reference Example 1

### (1) 3',5'-O-(1,1,3,3-Tetraisopropyldisiloxane-1,3-diyl)-adenosine

Adenosine (4.61 g, 17.3 mmol.) was subjected to azeotropic distillation with anhydrous pyridine. To the residue were added anhydrous pyridine (43 mL) and 1,1,3,3-tetraisopropyl-1,3-dichlorodisiloxane (5.70 mL, 1.72 mmol.). The resulting mixture was stirred at room temperature for 5 hours. Then, to the mixture were added dichloromethane (30 mL) and a mixture of pyridine and water (1:1, vol/vol, 20 mL). The organic layer was extracted three times with dichloromethane (30 mL x 3). The extracts were combined and placed under reduced pressure to remove the solvent. To the residue was added toluene, and the resulting solution was subjected to azeotropic distillation, to remove pyridine. The residual solution was placed on a silica gel column and eluted with 0.5% pyridine-methanol/ dichloromethane (1/50) to give the subject compound (8.30 g, 94%).

### (2) 2'-O-Tetrahydropyranyl-3',5'-O-(1,1,3,3-tetraisopropyldisiloxane-1,3-diyl)adenosine

In dichloromethane (65 mL) was dissolved 3',5'-O-(1,1,3^{,}3-tetraisopropyldisiloxane-1,3-diyl)adenosine (8.30 g, 16.29 mmol.). To the resulting solution was added dihydropyran (31.5 mL, 0.33 mol.), and then gradually added dropwise TFA (trifluoroacetic acid, 1.88 mL, 32.58 mmol.). The resulting mixture was allowed to stand for one day. Then, pyridine was added to the mixture to stop the reaction. Subsequently, the mixture was placed under reduced pressure to remove the solvent and TFA, and then extracted with dichloromethane-water. The organic layer is taken out, concentrated, placed on a silica gel column, and eluted with 0.5% pyridine-methanol/ dichloromethane (1/100) to isolate the subject compound (8.80 g, 91%).

### (3) 2'-O-Tetrahydropyranyl-N⁶-(N,N-dimethylaminomethylidene)adenosine

2'-O-Tetrahydropyranyl-3',5'-O-(1,1,3,3-tetraisopropyldisiloxane-1,3-diyl)adenosine (2.97 g, 5 mmol.) is dehydrated by subjecting it to azeotropic distillation with anhydrous pyridine. The dehydrated product was then dissolved in DMF (16 mL). To this solution was added an extremely excessive amount of N,N-dimethylformamide dimethylacetal (9.97 mL, 75 mmol.), and the resulting mixture was stirred at room temperature for 20 hours. After the reaction was complete, the solvent is distilled off, and the residue was dissolved in dichloromethane, washed sufficiently with water, and dried over anhydrous magnesium sulfate. The solvent was then distilled off, and the residue was dissolved in acetonitrile (50 mL). To the resulting solution were added potassium fluoride (1.74 g, 30 mmol.), tetraethylammonium bromide (6.30 g, 30 mmol.), and water (0.5 mL, 30 mmol.). The mixture was stirred at 50°C for one hour. After the reaction was complete, the mixture was cooled to room temperature, and insolubles were removed. Then, the solvent was distilled off, and the residue was dissolved in dichloromethane. A small amount of pyridine was incorporated into the dichloromethane solution, and the solution was washed with water. The aqueous layer was extracted five times with dichloromethane, and the organic extracts were combined and dried over anhydrous magnesium sulfate. The solvent was distilled off, and the residue was purified by chromatography using a silica gel column and eluting with methanol/dichloromethane (1/50) to give the subject compound (2.01 g, 99%).
¹H NMR (CDCl₃) δ ppm:
1.20-1.85 (m, 6H), 3.17 (d, J=4.0Hz, 6H), 5.67 (dd, J=7.0Hz, 3.0Hz, 1H), 7.77 (s, 1H), 8.37 (s, 1H), 8.80 (s, 1H).

### Reference Example 2

### (1) 2'-O-Tetrahydropyranyl-3',5'-O-(1,1,3,3-tetraisopropyldisiloxane-1,3-diyl)-N⁶-benzoyladenosine

2'-O-Tetrahydropyranyl-3',5'-O-(1,1,3,3-tetraisopropyldisiloxane-1,3-diyl)adenosine (2.47 g, 4.16 mmol.) prepared in the same manner as in Reference Example 1-(2) was subjected to azeotropic distillation with anhydrous pyridine. To the residue was added anhydrous pyridine (12 mL), and the mixture was sufficiently stirred. To the mixture was added benzoyl chloride (0.73 mL, 6.24 mmol.), and the resulting mixture was kept at room temperature. After 3 hours, ice was added to the reaction mixture to terminate the reaction. The reaction mixture was extracted using a mixture of methylene chloride and 10% aqueous NaHCO₃ solution. The organic layer was taken out, concentrated, and then subjected to azeotropic distillation with toluene to remove pyridine. The residue was placed on a silica gel column and eluted with 0.5% pyridine-CH₂Cl₂, to give the subject compound (2.44 g, 84%).
Analysis: Calculated(%) for C₃₄H₅₁N₅O₇Si₂·H₂O: C, 57.04; H, 7.46; N, 9.78: Found(%): C, 56.80; H, 7.58; N, 9.13.

### (2) 2'-O-Tetrahydropyranyl-N⁶-benzoyladenosine

In acetonitrile (53 mL) was dissolved 2'-O-tetrahydropyranyl-3',5'-O-(1,1,3,3-tetraisopropyldisiloxane-1,3-diyl)-N⁶-benzoyladenosine (1.86 g, 2.67 mmol.). To the resulting solution were added tetraethylammonium bromide (3.37 g, 16.0 mmol.), potassium fluoride (0.90 g, 16.0 mmol.), and water (0.8 mL). The mixture was then warmed at 50°C for 5 hours. The reaction mixture was filtered to remove the produced salt. The filtrate was placed under reduced pressure to distill off the solvent, and the residue was placed on a silica gel column and eluted with 0.5% pyridine and 2% methanol-CH₂Cl₂, to give the subject compound (1.19 g, 98%).
Analysis: Calculated(%) for C₂₂H₂₅N₅O₆: C, 58.02; H, 5.53; N, 15.38: Found(%): C, 57.65; H, 5.49; N, 15.52.

### EXAMPLE 1

### 2'-O-Tetrahydropyranyl-3',5'-O-di(9-phenylxanthenyl)-N⁶-(N,N-dimethylaminomethylidene)adenosine

2'-O-Tetrahydropyranyl-N⁶-(N,N-dimethylaminomethylidene)adenosine (3.66 g, 9 mmol.) was subjected several times to azeotropic distillation with anhydrous pyridine. The residue was then dissolved in anhydrous pyridine (90 mL). To the solution was added 9-phenylxanthenyl chloride (6.6 g, 22.5 mmol.), and the resulting mixture was stirred at room temperature for 12 hours. After the reaction was complete, a small amount of water was added to the reaction mixture to terminate the reaction. The solvent was then distilled off. The residue was dissolved in dichloromethane, washed with three portions of 5% aqueous sodium hydrogen carbonate solution, and dried over anhydrous magnesium sulfate. The solvent was then distilled off. The residue was purified by chromatography using a silica gel column and eluting with 0.5% triethylamine-dichloromethane/ n-hexane (6/4) to give the subject compound as an oil (5.31 g, 64%).
MS (FAB): 919 [M+1]
¹H NMR (CDCl₃) δ ppm:
1.15-1.69 (m, 6H), 3.14 (d, J=6.0Hz, 6H), 6.18 (d, J=6.0Hz, 1H), 6.55-7.45 (m, 26H), 7.80 (s, 1H), 8.28 (s, 1H), 8.70 (s, 1H).

### EXAMPLE 2

### 2'-O-Tetrahydropyranyl-3',5'-O-di(9-phenylxanthenyl)adenosine

In pyridine (100 mL) was dissolved 2'-O-tetrahydropyranyl-3',5'-O-di(9-phenylxanthenyl)-N⁶-(N,N-dimethylaminomethylidene)adenosine (5.31 g, 5.8 mmol.). To the resulting solution was added conc. aqueous ammonia (60 mL), and the mixture was stirred at room temperature for 5 hours. After the reaction was complete, the solvent was distilled off. The residue was dissolved in dichloromethane, washed with water, and dried over anhydrous magnesium sulfate. The solvent was then distilled off. The residue was purified by chromatography using a silica gel column and eluting with 0.5% triethylamine-dichloromethane/ n-hexane (7/3) to give the subject compound (4.77 g, 96%).
MS (FAB): 864 [M+1]
¹H NMR (CDCl₃) δ ppm:
1.05-1.72 (m, 6H), 5.71-5.95 (m, 1H), 6.02-6.26 (m, 2H), 6.55-7.50 (m, 20H), 7.70 (s, 1H), 8.10 (s, 1H).

### EXAMPLE 3

### 2'-O-Tetrahydropyranyl-3',5'-O-di[9-(4-methoxyphenyl)-xanthenyl]-N⁶-(N,N-dimethylaminomethylidene)adenosine

2'-O-Tetrahydropyranyl-N⁶-(N,N-dimethylaminomethylidene)adenosine (1.66 g, 4.01 mmol.) was subjected to azeotropic distillation with anhydrous pyridine. The residue was then dissolved in anhydrous pyridine (40 mL). To the solution was added 9-(4-methoxyphenyl)xanthenyl chloride (3.95 g, 12 mmol.), and the resulting mixture was stirred at room temperature for 12 hours. After the reaction was complete, a small amount of water was added to the reaction mixture to terminate the reaction. The solvent was then distilled off. The residue was dissolved in dichloromethane, washed with three portions of 5% aqueous sodium hydrogen carbonate solution, and dried over anhydrous magnesium sulfate. The solvent was then distilled off. The residue was purified by chromatography using a silica gel column and eluting with 1% triethylaminedichloromethane/n-hexane (7/3) to give the subject compound (3.1 g, 80%).
¹H NMR (CDCl₃) δ ppm:
1.00-1.80 (m, 6H), 3.15 (d, J=4.2Hz, 6H), 3.70 (d, J=4.2Hz, 6H), 6.18 (d, J=6.0Hz, 1H), 6.50-7.45 (m, 12H), 7.86 (s, 1H), 8.33 (s, 1H), 8.75 (s, 1H).

### EXAMPLE 4

### 2'-O-Tetrahydropyranyl-3',5'-O-di[9-(4-methoxyphenyl)xanthenyl]adenosine

In pyridine (100 mL) was dissolved 2'-O-tetrahydropyranyl-3',5'-O-di[9-(4-methoxyphenyl)xanthenyl]-N⁶-(N,N- dimethylaminomethylidene)adenosine (3.03 g, 3.1 mmol.). To the resulting solution was added conc. aqueous ammonia (60 mL), and the mixture was stirred at room temperature for 16 hours. After the reaction was complete, the solvent was distilled off. The residue was dissolved in dichloromethane, washed with water, and dried over anhydrous magnesium sulfate. The solvent was then distilled off. The residue was purified by chromatography using a silica gel column and eluting with 1% triethylamine-dichloromethane/ n-hexane(8/2) to give the subject compound (2.85 g, quantitative).

### EXAMPLE 5

### 2'-O-Tetrahydropyranyl-3',5'-O-di[9-(4-methoxyphenyl)-xanthenyl]-N³-(4-methoxybenzoyl)uridine

2'-O-Tetrahydropyranyl-N³-(4-methoxybenzoyl)uridine (3.36 g, 7.3 mmol.) was subjected to azeotropic distillation with anhydrous pyridine. The residue was then dissolved in anhydrous pyridine (70 mL). To the solution was added an excessive amount of 9-(4-methoxyphenyl)xanthenyl chloride, and the resulting mixture was stirred 4 hours. After the reaction was complete, a small amount of water was added to the reaction mixture to terminate the reaction. The solvent was then distilled off. The residue was dissolved in dichloromethane, washed with three portions of 5% aqueous sodium hydrogen carbonate solution, and dried over anhydrous magnesium sulfate. The solvent was then distilled off. The residue was purified by chromatography using a silica gel column and eluting with 1% triethylamine -dichloromethane/n-hexane (7/3) to give the subject compound.

### EXAMPLE 6

### 2'-O-Tetrahydropyranyl-3',5'-O-di(9-phenylxanthenyl]-N⁶-benzoyladenosine

2'-O-Tetrahydropyranyl-N⁶-benzoyladenosine (2.91 g, 6.41 mmol.) was subjected to azeotropic distillation with anhydrous pyridine. The residue was then dissolved in anhydrous pyridine (100 mL). To the solution was added an excessive amount of 9-phenylxanthenyl chloride, and the resulting mixture was stirred 4 hours. After the reaction was complete, a small amount of water was added to the reaction mixture to terminate the reaction. The solvent was then distilled off. The residue was dissolved in dichloromethane, washed with three portions of 5% aqueous sodium hydrogen carbonate solution, and dried over anhydrous magnesium sulfate. The solvent was then distilled off. The residue was purified by chromatography using a silica gel column and eluting with 0.5% triethylaminedichloromethane/n-hexane(6/4)-dichloromethane/methanol (50/1) to give the subject compound.

### PREPARATION EXAMPLE 1

### Tablets

The below-mentioned composition was granulated in an aqueous povidone solution, and to the granuates was added magnesium stearate and pressed to give a tablet.

| | |
|---|---|
| (1) Nucleoside derivative | 250 mg/tablet |
| (2) Lactose B.P. | 120 |
| (3) Povidone B.P. | 15 |
| (4) Sodium glycolated starch | 20 |
| (5) Magnesium stearate | 5 |
| | Total 500 mg/tablet |

### PREPARATION EXAMPLE 2

### Capsules

The below-mentioned composition was mixed and charged into a hard gelatin capsule to give a capsulated agent.

| | |
|---|---|
| (1) Nucleoside derivative | 250 mg/capsule |
| (2) Lactose B.P. | 143 |
| (3) Sodium glycolated starch | 25 |
| (4) Magnesium stearate | 2 |
| | Total 420 mg/capsule |

### PREPARATION EXAMPLE 3

### Parenteral solution

| | |
|---|---|
| (1) Nucleoside derivative | 0.20 g |
| (2) Benzyl alcohol | 0.10 |
| (3) Glycofurol | 1.45 |
| (4) Water for injection to dilute to give 3.00 mL. | |

The nucleoside derivative and benzyl alcohol were dissolved in glycofurol in order. To this solution was added water to adjust the volume to 3.00 mL. The resulting solution was filtered on a sterilyzed micropore filter and sealed in a sterilyzed amber vial.

## Claims

1. A nucleoside derivative having the formula (1-1) or (1-2): 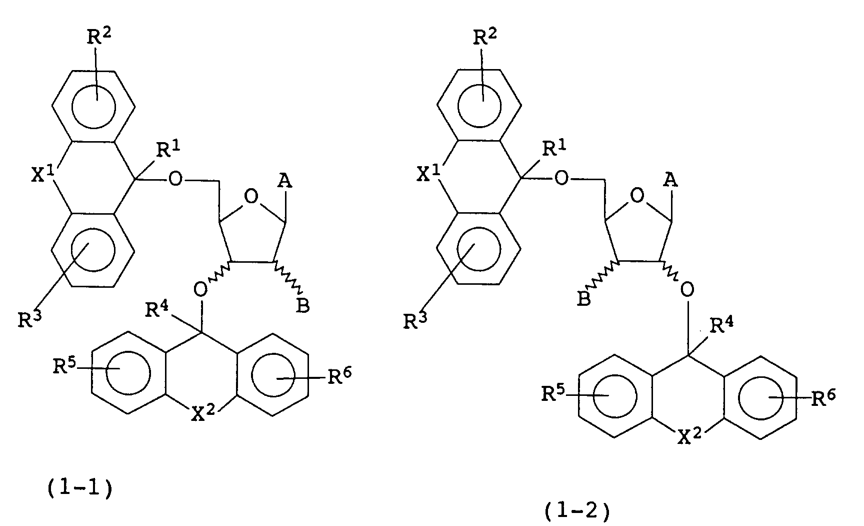 wherein A is a nitrogen atom-containing heterocyclic ring which is linked through nitrogen atom thereof; B is a hydrogen atom, a halogen atom, an azido group, an alkyl group having 1-7 carbon atoms, an aralkyl group having 5-19 carbon atoms, an alkoxyalkyl group having 2-15 carbon atoms, an alkoxy group having 1-7 carbon atom, a haloalkoxy group having 1-7 carbon atoms, an aralkyloxy group having 5-19 carbon atoms, an aryloxy group having 4-10 carbon atoms, a cycloalkyloxy group having 3-7 carbon atoms, or a cycloalkyloxy group having 4-6 carbon atoms and one or two heteroatoms selected from the group consisting of oxygen, nitrogen and sulfur; each of R¹ and R⁴ independently is a hydrogen atom, an alkyl group having 1-7 carbon atoms, a cycloalkyl group having 3-7 carbon atoms, an aralkyl group having 5-19 carbon atoms, or an aryl group having 4-10 carbon atoms, in which each of the aralkyl group and aryl group may have one or more substituents selected from the group consisting of a halogen atom, an alkyl group having 1-7 carbon atoms, an alkoxy group having 1-7 carbon atoms, a haloalkyl group having 1-7 carbon atoms, an alkoxyalkyl group having 2-15 carbon atoms and a haloalkoxy group having 1-7 carbon atoms; each of R², R³, R⁵ and R⁶ independently is a hydrogen atom, a halogen atom, a hydroxyl group, an alkyl group having 1-7 carbon atoms, an alkoxy group having 1-7 carbon atoms, a haloalkyl group having 1-7 carbon atoms, an alkoxyalkyl group having 2-15 carbon atoms, a haloalkoxy group having 1-7 carbon atoms, an aralkyl group having 5-19 carbon atoms, or an aryl group having 4-10 carbon atoms; and X¹ and X² independently is an oxygen atom, a sulfur atom, a methylene group or a carbonyl group, or its pharmaceutically acceptable salt.

2. The nucleoside derivative or its pharmaceutically acceptable salt as defined in claim 1, wherein R⁴, R⁵ and R⁶ in the formulas (1-1) and (1-2) are the same as R¹, R² and R³ in the formulas (1-1) and (1-2), respectively.

3. The nucleoside derivative or its pharmaceutically acceptable salt as defined in claim 1, wherein A in the formulas (1-1) and (1-2) is a group having one of the following formulas (5-1) to (5-4): 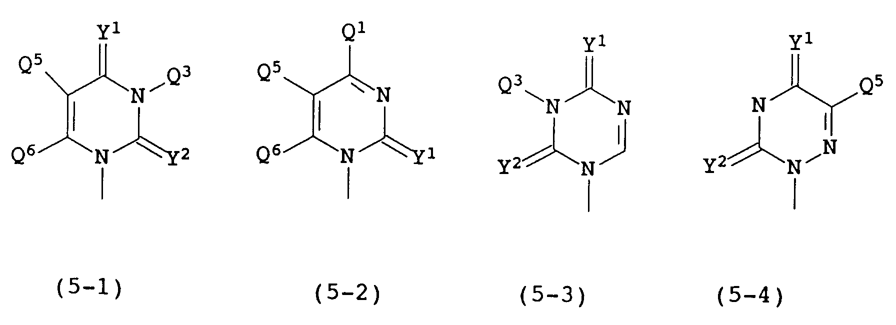 wherein
Q¹ is a hydrogen atom, a halogen atom, a group represented by -NQ⁷Q⁸, a group represented by -N=CQ⁹Q¹⁰, or a group represented by -OQ⁷, wherein each of Q⁷ and Q⁸ independently is a hydrogen atom, an alkyl group having 1-7 carbon atoms, a haloalkyl group having 1-7 carbon atoms, an aralkyl group having 5-19 carbon atoms, an aryl group having 4-10 carbon atoms, an acyl group having 1-10 carbon atoms, an allyl group, or a group having the formula (6): 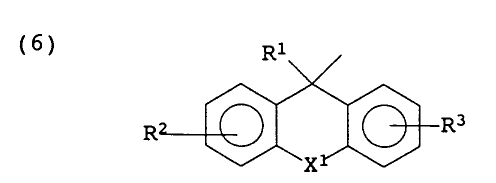 wherein R¹ is a hydrogen atom, an alkyl group having 1-7 carbon atoms, a cycloalkyl group having 3-7 carbon atoms, an aralkyl group having 5-19 carbon atoms, or an aryl group having 4-10 carbon atoms, wherein the aralkyl group and aryl group may have one or more substituents selected from the group consisting of a halogen atom, an alkyl group having 1-7 carbon atoms, an alkoxy group having 1-7 carbon atoms, a haloalkyl group having 1-7 carbon atoms, and a haloalkoxy group having 1-7 carbon atoms; each of R² and R³ independently is a hydrogen atom, a halogen atom, a hydroxyl group, an alkyl group having 1-7 carbon atoms, an alkoxy group having 1-7 carbon atoms, a haloalkyl group having 1-7 carbon atoms, a haloalkoxy group having 1-7 carbon atoms, an aralkyl group having 5-19 carbon atoms, or an aryl group having 4-10 carbon atoms; and X¹ is an oxygen atom, a sulfur atom, a methylene group, or a carbonyl group, and Q⁹ and Q¹⁰ independently is a hydrogen atom, an alkyl group having 1-7 carbon atoms, an alkoxy group having 1-7 carbon atoms, a haloalkyl group having 1-7 carbon atoms, an aralkyl group having 5-19 carbon atoms, an aryl group having 4-10 carbon atoms, an allyl group, or a group represented by -NQ⁷Q⁸ wherein each of Q⁷ and Q⁸ has the same meaning as above;
Q³ is a hydrogen atom, an alkyl group having 1-7 carbon atoms, a haloalkyl group having 1-7 carbon atoms, an aralkyl group having 5-19 carbon atoms, an acyl group having 1-10 carbon atoms, an aryl group having 4-10 carbon atoms, or an allyl group;
Q⁵ is a hydrogen atom, a halogen atom, an alkyl group having 1-7 carbon atoms, a haloalkyl group having 1-7 carbon atoms, an aralkyl group having 5-19 carbon atoms, an aryl group having 4-10 carbon atoms, or an allyl group;
Q⁶ is a hydrogen atom, a halogen atom, a thio group, or a seleno group, wherein the thio group and seleno group may have a substituent selected from the group consisting of an alkyl group having 1-7 carbon atoms, a haloalkyl group having 1-7 carbon atoms, an aralkyl group having 5-19 carbon atoms, an aryl group having 4-10 carbon atoms, or an allyl group; and
each of Y¹ and Y² independently is an oxygen atom or a sulfur atom.

4. The nucleoside derivative or its pharmaceutically acceptable salt as defined in claim 1, wherein A in the formulas (1-1) and (1-2) is a group having one of the following formulas (7-1) to (7-13): 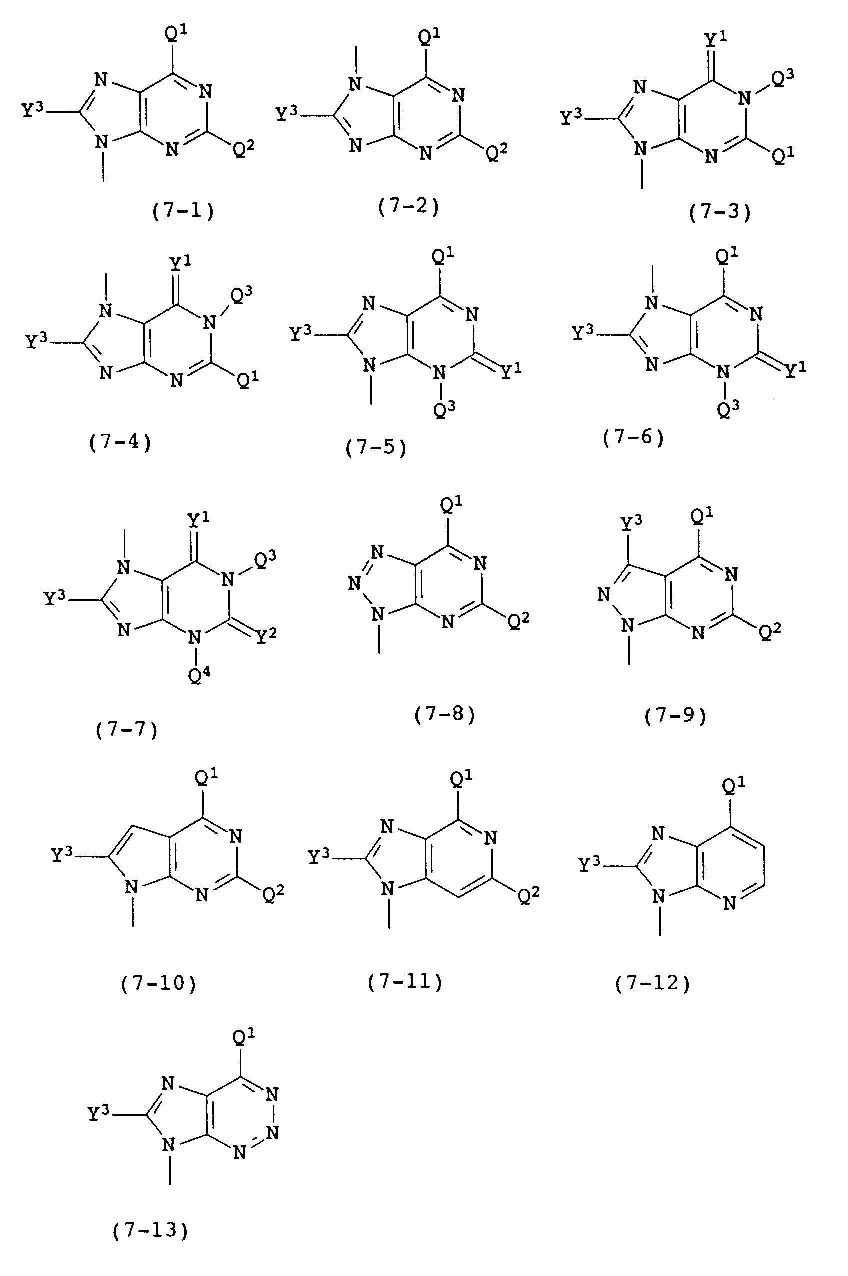 wherein each of Q¹, Q³, Y¹, and Y² has the same meaning as defined in claim 3, Q² has the same meaning as that of Q¹, Q⁴ has the same meaning as that of Q³, Y³ is a hydrogen atom, a halogen atom, or a hydroxyl group.

5. The nucleoside derivative or its pharmaceutically acceptable salt as defined in claim 1, wherein A in the formulas (1-1) and (1-2) is a group having one of the following formulas (8-1) to (8-27):

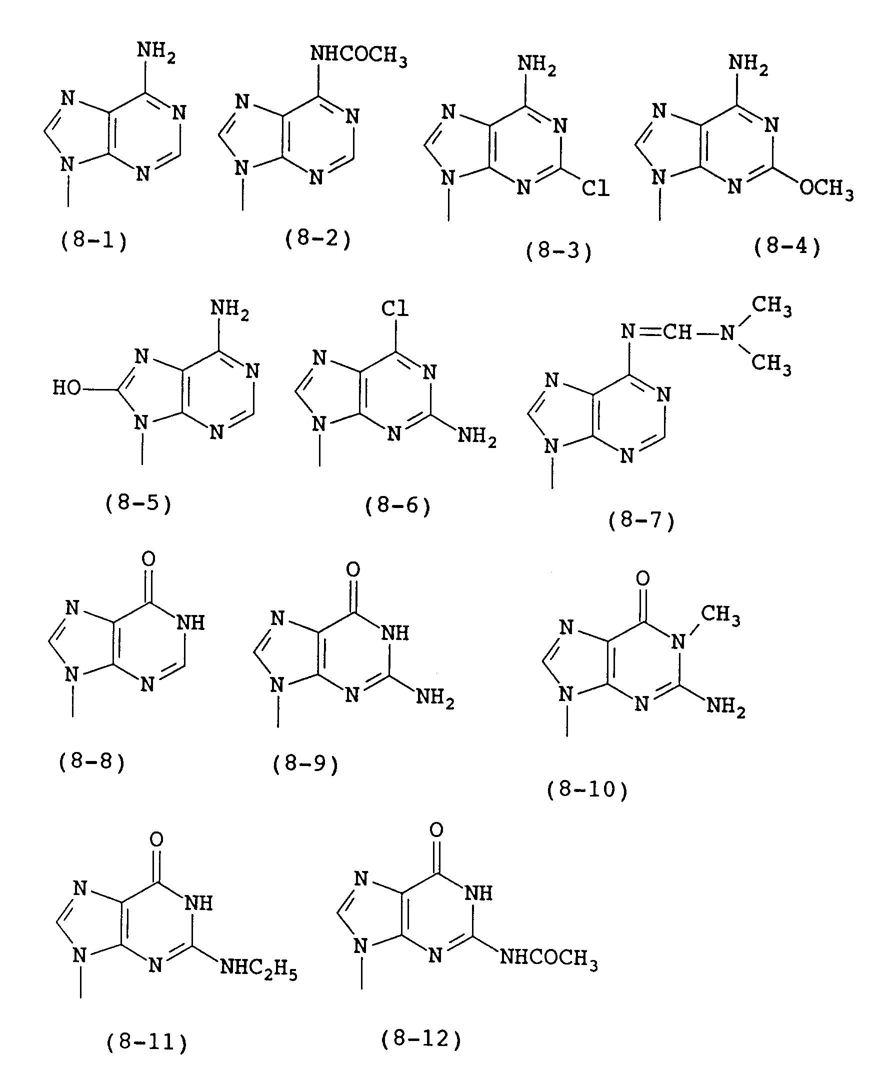

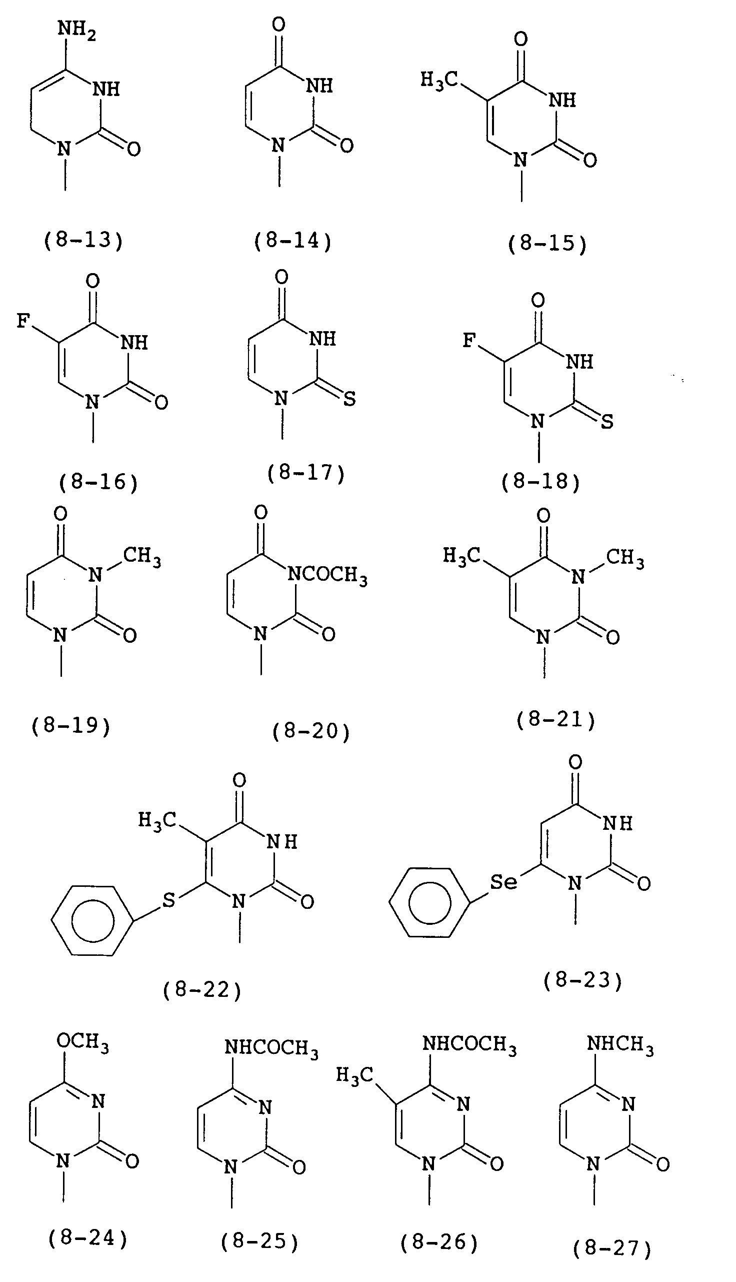

6. The nucleoside derivative or its pharmaceutically acceptable salt as defined in claim 1, wherein B in the formulas (1-1) and (1-2) is hydrogen, methoxy, ethoxy, phenoxy, trifluromethoxy, benzyloxy, fluorine, chlorine, bromine, azido, or one of the following formulas (9-1) to (9-6):

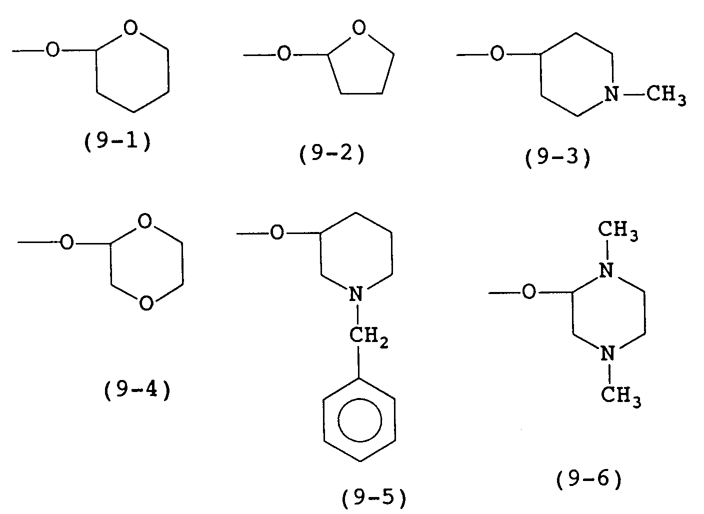

7. The nucleoside derivative or its pharmaceutically acceptable salt as defined in claim 1, wherein each of 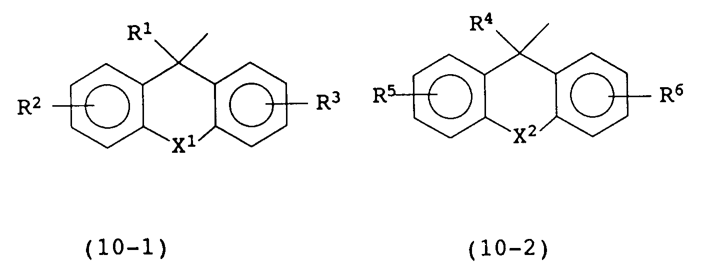 in the formulas (1-1) and (1-2) is one of the following groups:

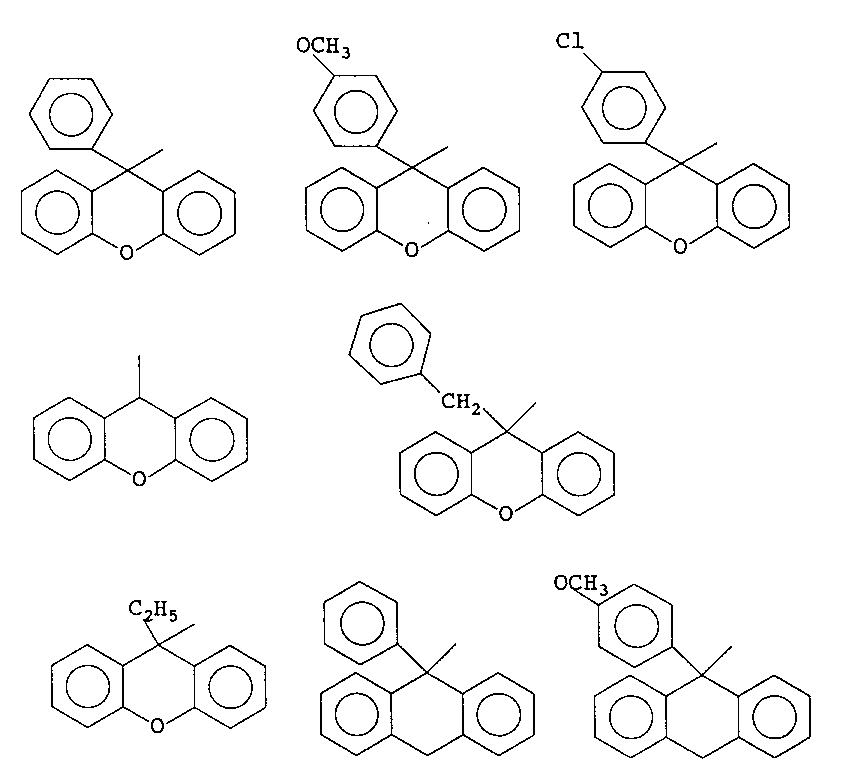

8. A process for the preparation of a nucleoside derivative as defined in claim 1 wherein R¹ and R⁴ are the same, R² and R⁵ are the same, and R³ and R⁶ are the same, which comprises causing a condensation reaction between a nucleoside derivative having the formula (2-1) or (2-2): 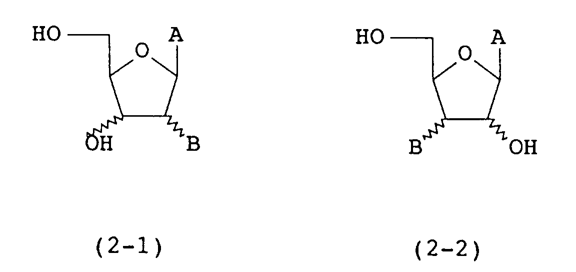 wherein each of A and B has the same meaning as defined in claim 1,
and a compound having the formula (3): 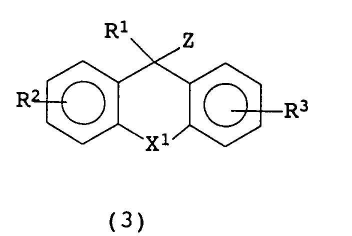 wherein each of R¹, R², R³, and X¹ has the same meaning as defined in claim 1, and Z is a halogen atom or a sulfonyloxy group.

9. An anti-herpes composition comprising the nucleoside derivative or its pharmaceutical salt as defined in claim 1 and a pharmaceutically acceptable additive.
